# EUROPEAN PATENT APPLICATION

(11) **EP 4 400 591 A1**
(43) Date of publication of application: **17.07.2024**
(21) Application number: 22867792.8
(22) Date of filing: 06.09.2022
(51) Int. Cl.: C12N 15/63, C12N 5/10, C12Q 1/6897, G01N 33/533

(54) **INDUCIBLE PROMOTER, VECTOR AND HOST CELL BASED THEREON**

(30) Priority: 07.09.2021 RU 2021126254
(71) Applicant: Joint Stock Company "Biocad", 198515, Saint Petersburg (RU)
(72) Inventor: KONONOV, Aleksey Vladimirovich, Moskovskaya obl., 142290 (RU); ZHIRIAKOVA, Mariia Vladimirovna, Moscow, 125183 (RU); GORDEEV, Aleksandr Andreevich, Moscow, 117405 (RU); PUCHKOVA, Mariia Yurievna, Tula, 300036 (RU); SOLOVYEV, Valery Vladimirovich, Moskovskaya obl., 142290 (RU); MOROZOV, Dmitry Valentinovich, Saint Petersburg, 190000 (RU)
(74) Representative: Meissner Bolte Partnerschaft mbB
(86) International application number: PCT/RU2022/050279
(87) International publication number: WO 2023/038547

(57) **Abstract**

The present invention relates to the fields of biotechnology and molecular biology, in particular to a universal inducible promoter, vector and host cell based thereon, as well as to a method for producing said host cell. The proposed inventions make it possible to analyze the activity of a target protein, for example, a receptor ligand, for example, a cytokine.

## Description

### Field of the invention

The present invention relates to the fields of biotechnology and molecular biology, in particular to a universal inducible promoter, vector and host cell based thereon, as well as to a method for producing said host cell. The proposed inventions make it possible to analyze the activity of a target protein, for example, a receptor ligand, for example, a cytokine.

### Background of the invention

Various therapeutically active polypeptides (for example, monoclonal antibodies, fusion proteins, various cytokines, receptor ligands, etc.) have proven their value as effective medicinal products for the treatment of a number of disorders and diseases.

To develop a new therapeutically active polypeptide, it is necessary to conduct functional tests to determine the biological activity of the given therapeutically active polypeptide.

Biological activity tests for a therapeutically active polypeptide are a set of techniques that are used to evaluate candidate medicinal products and that are based on analyzing the biological activity of a molecule in vitro using both primary cell cultures and specialty cell lines, or in vivo, using laboratory animals. Data from functional tests provide information on the activity of a candidate therapeutically active polypeptide.

Functional tests may be divided into the following groups:
1) test for specific activity (stimulation and inhibition of cell proliferation, cytotoxicity/apoptosis, antiviral activity, differentiation, migration, etc.);
2) test for reporter gene expression.

Proliferation/cytotoxicity regulation tests are the most universal tests for most therapeutically active polypeptides. These tests measure the level and activity of therapeutically active polypeptides via ability thereof to increase or decrease cell proliferation. The method is based on tracking the growth or death of cells in response to a test sample (Banks RE. Measurement of cytokines in clinical samples using immunoassays: problems and pitfalls. Crit Rev Clin Lab Sci. 2000 Apr;37(2):131-82. doi: 10.1080/10408360091174187. PMID: 10811142).

Such tests have found application in antiproliferation activity assays, for example, researchers evaluated the ability of IFN-β-1a to reduce the growth of WISH cells (Renato Mastrangeli ET AL., In vitro biological characterization of IFN-β-1a major glycoforms, Glycobiology, Volume 25, Issue 1, January 2015, Pages 21-29, https://doi.org/10.1093/glycob/cwu082).

Another notable example is a lymphocyte proliferation assay that is widely used to evaluate cellular immunity (Nikbakht, M. ET ALL, Evaluation of a new lymphocyte proliferation assay based on cyclic voltammetry; an alternative method. Sci Rep 9, 4503 (2019). https://doi.org/10.1038/s41598-019-41171-8). To date, cellular proliferation assays are commercially available for a plurality of cytokines as follows: IFN-γ, IL-1α, IL-1β, IL-2,3,4,5,6,7,8,10,13,15,19,21,33.

Reporter genes are typically used in cellular assays to track receptor-mediated expression changes at the transcription and/or translation levels. These optimized genes are expressed under the control of a sensitive element within a promoter, and the transcription factors bind thereto.

Phyllis A. Rees, R. Joel Lowy have reported the use of a reporter cell line to measure type 1 interferons, as an alternative to time- and labor-consuming proliferation assays (Phyllis A. ET ALL, Measuring type I interferon using reporter gene assays based on readily available cell lines, Journal of Immunological Methods, Volume 461, 2018, Pages 63-72, ISSN 0022-1759, https://doi.org/10.1016/j.jim.2018.06.007).

Jacqueline Mock et al. have developed a reporter line including a luciferase gene under the control of NF-kB-dependent promoter. An analogue was produced by conventional tests, such as CTLL-2 proliferation assay, ELISA to detect INF-γ induced IL-12, measurement of cytotoxic activity of TNF (Mock J. ET ALL, A universal reporter cell line for bioactivity evaluation of engineered cytokine products. Sci Rep. 2020;10(1):3234. Published 2020 Feb 24. doi:10.1038/s41598-020-60182-4).

Zeuner MT et al. have reported the use of a reporter cell line including NF-kB-sensitive promoter and a luciferase gene to investigate neuroinflammation. This assay makes it possible to evaluate the efficacy of pro-inflammatory molecules and peptides, as well as anti-inflammatory pharmaceuticals in nerve cells (Marie-Theres Zeuner ET ALL., Development and Characterisation of a Novel NF- κ B Reporter Cell Line for Investigation of Neuroinflammation, Mediators Inflamm, 2017, doi: 10.1155/2017/6209865).

Wang et al. have developed an analogue of a proliferation assay based on a cell line expressing luciferase under the control of SIE (serum response element) (Wang L. et al., Development of reporter gene assays to determine the bioactivity of biopharmaceuticals, Biotechnology Advances (2018), https://doi.org/10.1016/j.biotechadv.2019.107466).

Advantages of reporter cells when compared to conventional specific activity assays include the following:
1) easily reproducible results, automation and scaling;
2) specificity for a target medicinal product;
3) high level of sensitivity, low level of background activation;
4) ease of use, easy detection of reporter signal.

The disadvantages of reporter cells when compared to conventional specific activity assays include the long process of developing individual reporter cells for a target medicinal product, since it can last up to several months.

Thus, there is a need for reporter cells that could be used to determine the biological activity of a plurality of various therapeutically active polypeptides, rather than just one individual therapeutically active polypeptide.

### DESCRIPTION OF THE INVENTION

The authors of the invention have developed a universal inducible promoter comprising binding sites of transcription factors STAT3, STATS and AP-1 and a minimal promoter, as well as a vector and host cell based thereon, which make it possible to determine the biological activity of a plurality of various therapeutically active polypeptides, rather than just one individual therapeutically active polypeptide.

### Brief description of the invention

In one aspect, the present invention relates to an inducible promoter comprising binding sites of transcription factors STAT3, STATS and AP-1 and a minimal promoter.

In some embodiments of the invention, the inducible promoter comprises a binding site of the transcription factor STAT3, which is a nucleotide sequence selected from a group of nucleotide sequences including SEQ ID NO: 1, SEQ ID NO: 2 or SEQ ID NO: 3.

In some embodiments of the invention, the inducible promoter includes a binding site of the transcription factor STATS, which is a nucleotide sequence selected from a group of nucleotide sequences including SEQ ID NO: 4, SEQ ID NO: 5 or SEQ ID NO: 6.

In some embodiments of the invention, the inducible promoter comprises a binding site of the AP-1 family transcription factor, which is a nucleotide sequence selected from a group of nucleotide sequences including SEQ ID NO: 7, SEQ ID NO: 8 or SEQ ID NO: 9.

In some embodiments of the invention, the inducible promoter comprises binding sites of transcription factors STAT3, STATS and AP-1, which are arranged from 5' end to 3' end one after the other in the following order: STAT3-STAT5-AP-1.

In some embodiments of the invention, the inducible promoter comprises binding sites of transcription factors STAT3, STATS and AP-1, which are arranged from 5' end to 3' end one after the other in the following order: STAT3-STAT5-AP-1 and comprise a nucleotide sequence selected from a group of nucleotide sequences including SEQ ID NO: 10, SEQ ID NO: 11 or SEQ ID NO: 12.

In some embodiments of the invention, the inducible promoter comprises binding sites of transcription factors STAT3, STATS and AP-1, which include the nucleotide sequence of SEQ ID NO: 13.

In some embodiments of the invention, the inducible promoter comprises a minimal promoter comprising a TATA box.

In some embodiments of the invention, the inducible promoter comprises a minimal promoter comprising the nucleotide sequence of SEQ ID NO: 14.

In one aspect, the present invention relates to an inducible expression vector comprising, in 5' end to 3' end direction, any of the above inducible promoters and a reporter gene.

In some embodiments of the invention, the inducible expression vector comprises a reporter gene being a gene of firefly luciferase protein.

In some embodiments of the invention, the inducible expression vector comprises a gene of firefly luciferase protein, which comprises the nucleotide sequence of SEQ ID NO: 15.

In some embodiments of the invention, the inducible expression vector comprises the nucleotide sequence of SEQ ID NO: 16.

In one aspect, the present invention relates to a method for producing a host cell for analyzing the activity of a target protein, said method comprising transforming the cell with any of the above inducible expression vectors.

In one aspect, the present invention relates to a host cell for analyzing the activity of a target protein, said host cell comprising any of the above inducible promoters and a reporter gene.

In some embodiments of the invention, the host cell comprises a reporter gene being a gene of firefly luciferase protein.

In some embodiments of the invention, the host cell comprises a gene of firefly luciferase protein, which comprises the nucleotide sequence of SEQ ID NO: 15.

In some embodiments of the invention, the host cell comprises the nucleotide sequence of SEQ ID NO: 16.

In some embodiments of the invention, the host cell is used to analyze the activity of a target protein, wherein the target protein is a receptor ligand.

In some embodiments of the invention, the host cell is used to analyze the activity of a target protein, wherein the target protein is a cytokine.

### Brief description of drawings

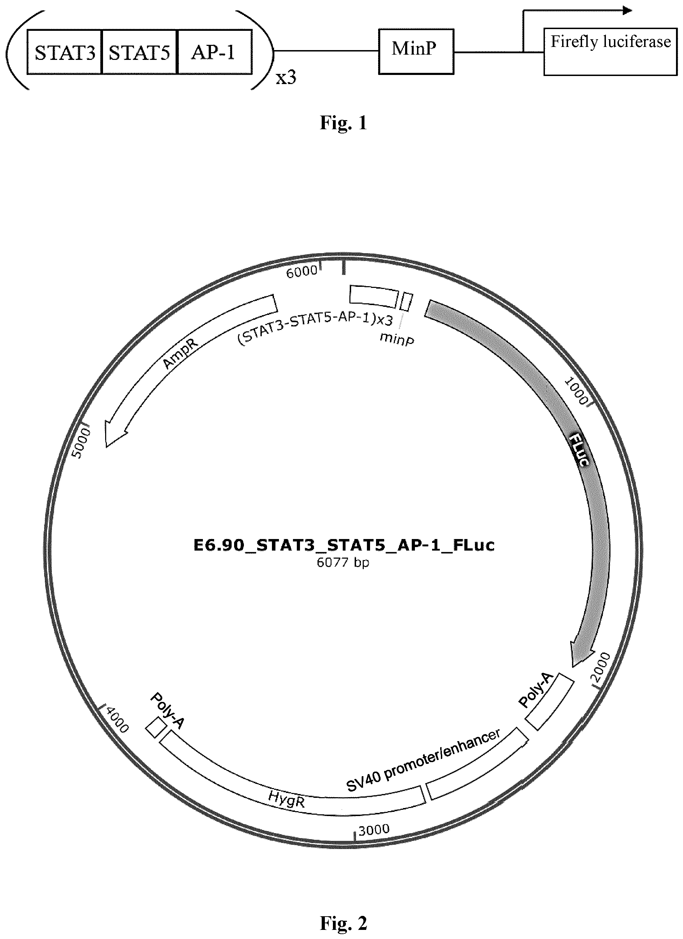

Fig. 1 is a diagram of genetic reporter construct. This construct comprises the nucleotide sequence of the reporter firefly luciferase protein gene under the control of STAT3, STATS, AP-1-dependent promoter. The non-coding region includes a minimal promoter consisting of a TATA box, at the 5' end of which there is positioned a cis-regulatory element consisting of alternating binding sites of the transcription factors STAT3, STATS, AP-1 in three repeats, as indicated in the diagram. The flanking sequences are complementary to the regions surrounding the expression cassette within the target vector and comprise recognition sites for restriction endonucleases Acc65I and BglII.

FIg. 2 is a map of the reporter plasmid vector E6.90_STAT3_STAT5_AP-1_FLuc encoding the firefly luciferase gene under the control of STAT3, STATS, AP-1-dependent promoter.
AmpR is a betalactamase gene that conveys resistance to ampicillin, and allows selection in E.coli cells;
Poly-A is a polyadenylation signal, transcription terminator;
STAT3-STAT5-AP-1 is a regulatory element regulating FLuc gene expression;
MinP is a minimal promoter comprising a TATA box;
FLuc is the Photinus pyralis (firefly) luciferase gene;
HygR is a gene that conveys resistance to hygromycin. It enables selection in eukaryotic cell culture;
SV40 promoter/enhancer is a eukaryotic promoter/enhancer of the SV40 virus.

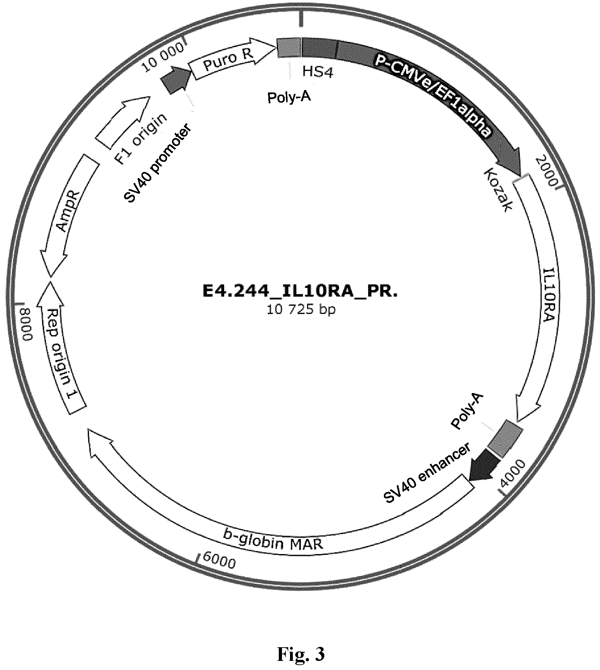

Fig. 3 is a map of the expression plasmid vector E4.244IL10RA PR encoding the human IL10RA gene.
HS4 is the HS4 insulator;
pCMVe/EF 1 alpha is a constitutive hybrid promoter of CMV/EF1alpha;
Kozak is a Kozak sequence;
IL10RA is the human IL10RA gene;
Poly-A is a polyadenylation signal, transcription terminator;
SV40 enhancer is an enhancer of the SV40 virus;
b-globin MAR is a MAR-element (M5) of the human b-globin gene;
Rep origin 1 is a replication origin site;
AmpR is a beta-lactamase gene that provides resistance to ampicillin. It enables selection in E.coli cell culture;
F1 origin is bacteriophage f1 replication origin;
SV40 promoter is a eukaryotic promoter of the SV40 virus.

Puro R is eukaryotic resistance to puromycin. The gene that conveys resistance to puromycin. It enables selection in eukaryotic cell culture.

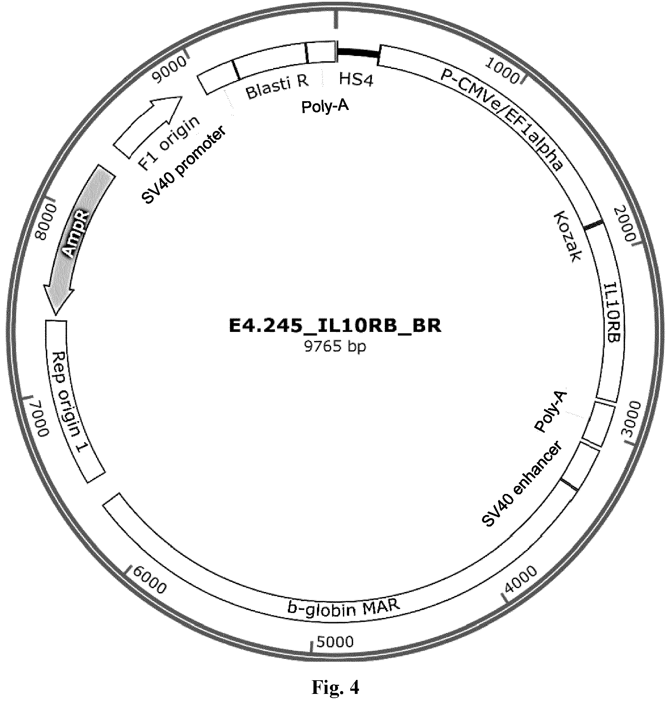

Fig. 4 is a map of the expression plasmid vector E4.245_IL10RB_BR encoding the human IL10RB gene.
HS4 is the HS4 insulator;
pCMVe/EF1alpha is a constitutive hybrid promoter of CMV/EF1alpha;
Kozak is a Kozak sequence;
IL10RA is the human IL10RA gene;
Poly-A is a polyadenylation signal, transcription terminator;
SV40 enhancer is an enhancer of the SV40 virus;
b-globin MAR is a MAR-element (M5) of the human b-globin gene;
Rep origin 1 is a replication origin site;
AmpR is a beta-lactamase gene that provides resistance to ampicillin. It enables selection in E.coli cell culture;
F1 origin is bacteriophage f1 replication origin;
SV40 promoter is a eukaryotic promoter of the SV40 virus;
Blasti R is eukaryotic resistance to Blasticidin. The gene that conveys resistance to Blasticidin. It enables selection in eukaryotic cell culture.

Fig. 5 is a histogram showing the multiplicity of activation of HEK293 Nf-kB reporter cells in response to different doses of PMA.

Fig. 6 is a histogram showing the multiplicity of activation of the reporter clonal lines HEK293_STAT3-STAT5-AP1_FLuc in response to PMA concentration of 33nM.
A2, A4, A5, B1, B3, B5, B6, C4, C6, D1, D4, C4#2, B0>, B1>, B2>, B3> are names of various clonal reporter lines;
initial - untransfected parent cells;
pool - cell population following transfection.
Pool and untransfected cells were used as controls.

Fig. 7 is a histogram showing the multiplicity of activation of the reporter clonal lines HEK293_STAT3-STAT5-AP1_FLuc expressing IL10R in response to IL10 at 100 ng/ml.

A1.2, A1.4, A1.5, A1.7, C1.1, C1.2, C1.6, C1.15, C1.11, C1.14, C1.16, A4_1, A4_3, A4_9, A4_10, C4_3, C4_12, C4_13, A5, A8, A9, A12, A15, A23, A31, C44, C4.4, C12, C16, C20, C27, C28, C29, C31, C33, C40, C41 are names of various clonal lines of the IL10 reporter.

Figure 8 is a graph showing the dose-dependent response of HEK293-STAT3-STATS-AP1_FLuc_IL10R cells to IL10 activation.

Fig. 9 is a graph of GAS6-dependent activation of HEK293_STAT3-STAT5-AP1 FLuc AXL pools produced on the basis of three different clones (Cl. D1, cl. B3, cl. C4#2) of the reporter cell line HEK293_STAT3-STAT5-AP1_FLuc. GAS6 is an activator of AXL-dependent cell signaling.

Cl. D1, cl. B3, cl. C4#2) are clones of the reporter cell line HEK293_STAT3-STAT5-AP1_FLuc.

Fig. 10 is a graph showing activation of the reporter cell line HEK293_STAT3-STAT5-AP1_FLuc_AXL as a function of concentration of various GAS6 products.

GAS6 product #1, #2, #3 are three different Gas6 products.

Fig. 11 is a graph showing activation of the reporter cell line HEK293AXL_STAT3-STAT5-AP1 as a function of cell incubation time in the presence of GAS6.

Fig. 12 is a graph showing DU145 cell proliferation as a function of GAS6 concentration.

Fig. 13-15 are graphs showing GAS6-dependent activation of various clones (clone C2 in Figure 13, clone H4 in Figure 14, clone E8 in Figure 15) of DU145_STAT3-STAT5-AP1_FLuc.

Clones C2, H4 and E8 are clones of the DU145_STAT3-STAT5-AP1 cell line produced on the basis of DU145 cell line, a natural AXL expresser, using a construct encoding firefly luciferase under the control of the STAT3-STAT5-AP1 promoter. These clones showed the highest response levels to GAS6 processing.

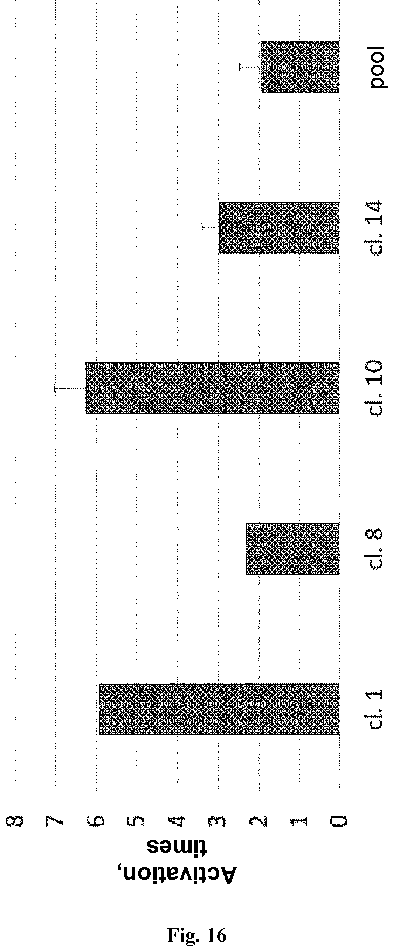

Figure 16 is a graph of GAS6-dependent activation of the pool and individual clones of HEK293_AXL_STAT3-STAT5-AP1_FLuc.

Cl. 1, cl. 8, cl. 10, cl. 14 are clones of the HEK293AXL_STAT3-STAT5-AP1_FLuc cell line, which were produced from the HEK293AXL_STAT3-STATS-AP1_FLuc pool.

Pool is the cell line HEK293_AXL_STAT3-STAT5-AP1_FLuc produced from the cell line HEK293 AXL, an AXL overexpresser, into which a gene encoding firefly luciferase was introduced under the control of STAT3-STAT5-AP1-dependent promoter.

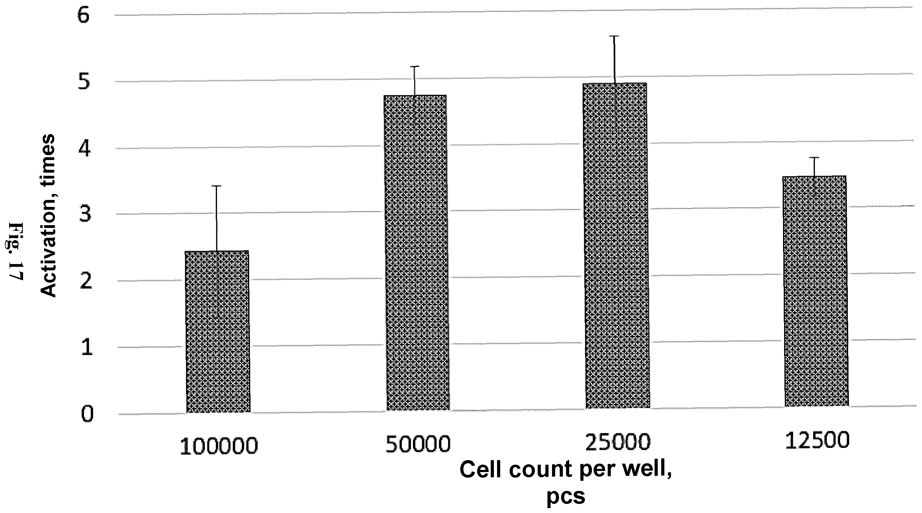

Fig. 17 is a graph showing activation of the reporter cell line HEK293_AXL_STAT3-STAT5-AP1 as a function of cell count.

### Definitions and general methods

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms. Typically, the present classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known by those skilled and widely used in the art. Enzyme reactions and purification methods are performed according to the manufacturer's guidelines, as is common in the art, or as described herein.

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

As used in the present description, polynucleotides include, as non-limiting examples, all nucleic acid sequences which are obtained by any means available in the art, including, as non-limiting examples, recombinant means, i.e. the cloning of nucleic acid sequences from a recombinant library or a cell genome, using ordinary cloning technology and PCR and the like, and by synthetic means.

It should also be included here that the present invention does not relate to nucleotide sequences in their natural chromosomal environment, i.e. in a natural state. The sequences of the present invention have been isolated and/or purified, i.e., they were sampled directly or indirectly, for example by copying, their environment having been at least partially modified. Thus, isolated nucleic acids obtained by recombinant genetics, by means, for example, of host cells, or obtained by chemical synthesis should also be mentioned here.

Unless otherwise indicated, the term nucleotide sequence encompasses its complement. Thus, a nucleic acid having a particular sequence should be understood as one which encompasses the complementary strand thereof with the complementary sequence thereof.

The term "vector" as used herein means a nucleic acid molecule capable of transporting another nucleic acid to which it has been linked.

As used in the present description, the term "expression" is defined as the transcription and/or translation of a particular nucleotide sequence driven by its promoter.

### Detailed description of the invention

### Inducible promoter

In one aspect, the present invention relates to an inducible promoter comprising binding sites of transcription factors STAT3, STATS and AP-1 and a minimal promoter.

The inducible promoter according to the invention is universal for use in reporter cell systems to determine the activity of receptor ligands, for example, cytokines.

Inducible promoter refers to a promoter that provides gene expression in response to a specific signal.

The authors of the invention have surprizingly found that the presence of binding sites of transcription factors STAT3, STATS and AP-1 in the inducible promoter makes it possible to determine the level of expression of the reporter gene in response to a specific signal produced from the interaction between the ligand and receptor thereof, wherein the receptor ligand may be selected from a plurality of cytokines and other receptor ligands comprising: IL-2, IL-4, IL-7, IL-9, IL-13, IL-15, IL-21, GM-CSF, IL-3, IL-5, IL-6, IL-11, IL-12, IL-27, LIF, OSM, CNTF, CT-1, CLC, NP, leptin, NNT-1BSF-3, IFN-α, IFN- β, IFN-γ , IL-23, IL-10 , IL-20 , IL-22, IL-28, IL-29, IL-19, IL-24, IL-26, IL37, IL18, M-CSF, CSF1, G-CSF, CSF 3, IL-31, IL-35, growth hormone, prolactin, THPO, MGDF, EPO, TSLP, TNFSF18, IL-1, IL-17, TNF, TNF-α, RANKL (OPGL/TRANCE), TRAIL, VEGI, CD153 (CD30L), CD154 (CD40L), CD70 (CD27L), TNF-C, CD70, 4-1BB ligand or GAS6, thus making said inducible promoter universal for use in reporter cell systems to determine the activity of ligands to the receptor, for example, cytokines.

The binding site of the transcription factor STATS comprises a core portion being TTCnnnGAA.

In some embodiments of the invention, the inducible promoter comprises a binding site of the transcription factor STAT3, which is a nucleotide sequence selected from a group of nucleotide sequences including SEQ ID NO: 1, SEQ ID NO: 2 or SEQ ID NO: 3.

The binding site of the transcription factor STATS comprises a core portion being TTCnnnGAA.

In some embodiments of the invention, the inducible promoter includes a binding site of the transcription factor STATS, which is a nucleotide sequence selected from a group of nucleotide sequences including SEQ ID NO: 4, SEQ ID NO: 5 or SEQ ID NO: 6.

The binding site of the transcription factor AP-1 includes a core portion being T[G/T]A[C/G]TCA.

In some embodiments of the invention, the inducible promoter comprises a binding site of the AP-1 family transcription factor, which is a nucleotide sequence selected from a group of nucleotide sequences including SEQ ID NO: 7, SEQ ID NO: 8 or SEQ ID NO: 9.

In some embodiments of the invention, the inducible promoter comprises binding sites of transcription factors STAT3, STATS and AP-1, which are arranged from 5' end to 3' end one after the other in the following order: STAT3-STAT5-AP-1.

In some embodiments of the invention, the inducible promoter includes binding sites of transcription factors STAT3, STATS and AP-1, which are arranged from 5' end to 3' end one after the other in the following order: STAT3-STAT5-AP-1, wherein STAT3-STAT5-AP-1 is represented in 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more repeats. The maximum amount of STAT3-STAT5-AP-1 repeats is limited by the capacity of the expression vector. The amount of STAT3-STAT5-AP-1 repeats does not effect the performance of the inducible promoter according to the invention.

In some embodiments of the invention, the inducible promoter comprises binding sites of transcription factors STAT3, STATS and AP-1, which are arranged from 5' end to 3' end one after the other in the following order: STAT3-STAT5-AP-1 and comprise a nucleotide sequence selected from a group of nucleotide sequences including SEQ ID NO: 10, SEQ ID NO: 11 or SEQ ID NO: 12.

In some embodiments of the invention, the inducible promoter comprises binding sites of transcription factors STAT3, STATS and AP-1, which include the nucleotide sequence of SEQ ID NO: 13.

In some embodiments of the invention, the inducible promoter comprises a minimal promoter comprising a TATA box.

Minimal promoter refers to a promoter with lowest regulatory element content, comprising a TATA box (Hogness box) and a transcription initiation site; the promoter will not function unless special regulatory elements are added in front of same.

In some embodiments of the invention, the inducible promoter comprises a minimal promoter comprising the nucleotide sequence of SEQ ID NO: 14.

### Inducible expression vector

In one aspect, the present invention relates to an inducible expression vector comprising, in 5' end to 3' end direction, any of the above inducible promoters and a reporter gene.

The inducible expression vector according to the invention is universal for producing reporter cell systems to determine the activity of ligand to receptor thereof, for example, a cytokine.

The inducible expression vector is a recombinant vector.

In some embodiments of the invention, the inducible expression vector comprises a reporter gene being a gene of luciferase protein.

In some embodiments of the invention, the inducible expression vector comprises a reporter gene being a gene of firefly luciferase protein.

In some embodiments of the invention, the inducible expression vector comprises a gene of firefly luciferase protein, which comprises the nucleotide sequence of SEQ ID NO: 15.

In some embodiments of the invention, the inducible expression vector comprises the nucleotide sequence of SEQ ID NO: 16.

In some embodiments of the invention, the inducible expression vector comprises a reporter gene being a gene encoding green fluorescent protein (GFP), or a red fluorescent protein gene (DsRed), or a LacZ gene encoding the enzyme beta-galactosidase, or any other gene encoding fluorescent or luminescent proteins that may be used as a reporter gene.

In some embodiments of the invention, the inducible expression vector comprises a heterologous nucleic acid sequence comprising regulatory sequences that provide for the expression of the gene of reporter polypeptide.

"Regulatory sequences that provide the expression of product encoded by the heterologous nucleic acid sequence in target cells", as used in the present invention, mean polynucleotide sequences that are necessary to influence the expression and processing of coding sequences to which they are cloned. Expression control sequences include appropriate transcription initiation, termination, inducible promoter and enhancer sequences; efficient RNA processing signals such as splicing and polyadenylation signals; sequences that stabilize cytoplasmic mRNA; sequences that enhance translation efficiency (i.e. Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "regulatory sequences" encompasses at least all components whose presence is important for expression and processing.

The terms "enhancers" or "enhancer" as used herein may refer to a DNA sequence that is located adjacent to the DNA sequence that encodes a recombinant product. Enhancer elements are typically located in a 5' direction from a promoter element or can be located downstream of or within a coding DNA sequence (e.g. a DNA sequence transcribed or translated into a recombinant product or products). Hence, an enhancer element may be located 100 base pairs, 200 base pairs, or 300 or more base pairs upstream of a DNA sequence that encodes a recombinant product, or downstream of said sequence. Enhancer elements may increase the amount of a recombinant product being expressed from a DNA sequence above the level of expression associated with a single promoter element. Multiple enhancer elements are readily available to those of ordinary skill in the art.

In some embodiments of the invention, the universal inducible expression vector may be used to generate a host cell that is used as a universal reporter cell line to analyze the activity of a plurality of various receptor ligands, which, for example, are IL-2, IL-4, IL-7, IL-9, IL-13, IL-15, IL-21, GM-CSF, IL-3, IL-5, IL-6, IL-11, IL-12, IL-27, LIF, OSM, CNTF, CT-1, CLC, NP, leptin, NNT-1/BSF-3, IFN-α, IFN-β, IFN-γ , IL-23, IL-10 , IL-20 , IL-22, IL-28, IL-29, IL-19, IL-24, IL-26, IL37, IL18, M-CSF, CSF1, G-CSF, CSF 3, IL-31, IL-35, growth hormone, prolactin, THPO, MGDF, EPO, TSLP, TNFSF18, IL-1, IL-17, TNF, TNF-α, RANKL (OPGL/TRANCE), TRAIL, VEGI, CD153 (CD30L), CD154 (CD40L), CD70 (CD27L), TNF-C, CD70, 4-1BB ligand or GAS6.

### Host cell and method for producing same

In one aspect, the present invention relates to a method for producing a host cell for analyzing the activity of a target protein, said method comprising transforming the cell with any of the above inducible expression vectors.

In one aspect, the present invention relates to a host cell for analyzing the activity of a target protein, said host cell comprising any of the above inducible promoters and a reporter gene.

Due to the features of the above inducible promoters according to the invention, the host cell according to the invention may be used to analyze the activity of a plurality of various receptor ligands, which, for example, are IL-2, IL-4, IL-7, IL-9, IL-13, IL-15, IL-21, GM-CSF, IL-3, IL-5, IL-6, IL-11, IL-12, IL-27, LIF, OSM, CNTF, CT-1, CLC, NP, leptin, NNT-1BSF-3, IFN-α, IFN- β, IFN-γ , IL-23, IL-10 , IL-20 , IL-22, IL-28, IL-29, IL-19, IL-24, IL-26, IL37, IL18, M-CSF, CSF1, G-CSF, CSF 3, IL-31, IL-35, growth hormone, prolactin, THPO, MGDF, EPO, TSLP, TNFSF18, IL-1, IL-17, TNF, TNF-α, RANKL (OPGL/TRANCE), TRAIL, VEGI, CD153 (CD30L), CD154 (CD40L), CD70 (CD27L), TNF-C, CD70, 4-1BB ligand or GAS6.

The host cell according to the invention is a recombinant host cell.

In some embodiments of the invention, the host cell comprises a reporter gene being a gene of luciferase protein.

In some embodiments of the invention, the host cell comprises a reporter gene being a gene of firefly luciferase protein.

In some embodiments of the invention, the host cell comprises a gene of firefly luciferase protein, which comprises the nucleotide sequence of SEQ ID NO: 15.

In some embodiments of the invention, the host cell comprises the nucleotide sequence of SEQ ID NO: 16.

In some embodiments of the invention, the host cell comprises a reporter gene being a gene encoding green fluorescent protein (GFP), or a red fluorescent protein gene (DsRed), or a LacZ gene encoding the enzyme beta-galactosidase, or any other gene encoding fluorescent or luminescent proteins that may be used as a reporter gene.

In some embodiments of the invention, the host cell is used to analyze the activity of a target protein, wherein the target protein is a receptor ligand.

In some embodiments of the invention, the host cell is used to analyze the activity of a target protein, wherein the target protein is a cytokine.

In some embodiments of the invention, the host cell is a eukaryotic cell.

In some embodiments of the invention, the host cell is a mammalian cell.

Mammalian cell lines used as hosts for transformation are well known in the art and include a plurality of immortalized cell lines available. These include, e.g., Chinese hamster ovary (CHO) cells, NS0 cells, SP2 cells, HEK-293T cells, FreeStyle 293 cells (Invitrogen), NIH-3T3 cells, HeLa cells, baby hamster kidney (BHK) cells, African green monkey kidney cells (COS), human hepatocellular carcinoma cells (e.g., Hep G2), A549 cells, and a number of other cell lines. Cell lines are selected by determining which cell lines have high expression levels and provide for necessary characteristics of the protein produced. Other cell lines that may be used are insect cell lines, such as Sf9 or Sf21 cells. Plant host cells include e.g. Nicotiana, Arabidopsis, duckweed, corn, wheat, potato, etc. Bacterial host cells include Escherichia and Streptomyces species. Yeast host cells include Schizosaccharomyces pombe, Saccharomyces cerevisiae and Pichia pastoris.

The above host cell of the invention does not refer to a host cell produced using human embryos.

The above host cell of the invention does not refer to a host cell produced by modifying the genetic integrity of human germline cells.

### Examples

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

### Materials and general methods

### Recombinant DNA techniques

Standard methods were used to manipulate DNA as described in Sambrook, J. et al, Molecular cloning: A laboratory manual; Cold Spring Harbor Laboratory Press, Cold Spring Harbor, New York, 1989. The molecular biological reagents were used according to the manufacturer protocols.

### Gene synthesis

Desired gene segments were prepared from oligonucleotides made by chemical synthesis. The gene segments of 300-1400 bp long, which were flanked by singular restriction sites, were assembled by annealing and ligation of oligonucleotides including PCR amplification and subsequently cloned via the restriction sites. The DNA sequences of the subcloned gene fragments were confirmed by DNA sequencing.

### DNA sequence determination

DNA sequences were determined by Sanger sequencing.
DNA sequence analysis and sequence data management

The Unipro's UGENE suite version 1.29 and SnapGene Viewer were used for sequence creation, mapping, analysis, annotation and illustration.

### Expression vectors

The vectors comprising the reporter gene, as described in the application materials, comprised, in addition to the reporter construct: a replication initiation site that provides for replication of said plasmid in E.coli, a gene that conveys E.coli resistance to a selective antibiotic (ampicillin).

The genetic constructs of the vector as described below were assembled by PCR and/or gene synthesis and assembly using known recombinant methods and techniques by connection of the according nucleic acid segments, e.g. using unique restriction sites in the corresponding vectors. The subcloned nucleic acid sequences were verified by DNA sequencing. For transient transfections, larger quantities of the plasmids were prepared by plasmid preparation from transformed E. coli cultures.

### Example 1. Production of reporter plasmid vector encoding firefly luciferase gene under the control of STAT3-STATS-AP-1-sensitive promoter.

To generate a universal reporter construct, we selected the transcription factors STAT3, STATS, AP-1 mediating the activity of many various signaling molecules.

We created a regulatory element including a minimal promoter consisting of a TATA box, and at 5' end of the promoter there were positioned binding sites for the transcription factors STAT3, STATS, AP-1 (doi:10.1128/JVI.01713-10; doi:10.1074/jbc.M001748200) in three repeats (Fig. 1). This combination of transcription factor binding sites makes it possible to produce an inducible promoter having a vast scope of applications.

To assemble the array, we synthesized six partially complementary oligonucleotides of 60 bp long, with 20 bp overlap. The assembly was carried out by synthesis from oligonucleotides, resulting in a fragment of 249 bp long, flanked by regions complementary to the plasmid vector. The resulting sequence was cloned within the vector E6.90 by ligation independent cloning (Fig. 2). The cloned nucleic acid sequences were verified by DNA sequencing.

### Example 2. Production of plasmid vectors encoding IL-10 receptor subunits to create a stable expresser cell line.

To evaluate the reporter line in a specific cell test, we expressed the IL-10 receptor onto the surface of the reporter cells.

To generate native sequences of the human IL-10Ra gene (https://www.uniprot.org/uniprot/Q13651) and the human IL-10Rb gene (https://www.uniprot.org/uniprot/Q08334), we isolated total RNA from donor's peripheral blood mononuclear cells and synthesized cDNA on the basis of same. The resulting cDNA library was used as a template for PCR amplification of target sequences flanked by restriction sites. Target genes within expression vectors were cloned by the restriction-ligation method (Fig. 3, Fig. 4). The cloned nucleic acid sequences were verified by DNA sequencing.

### Example 3. Activation of HEK293 Nf-kB.

We used a PMA activator to screen clonal reporter lines. This agent directly effects protein kinase C, thus triggering signaling cascades that activate the reporter gene expression (https://doi.org/10.1007/s11373-005-1210-5; doi: 10.1093/carcin/bgm261). To determine the optimal dose of PMA, we conducted experiments involving activation of the HEK293 Nf-kB reporter line. Cells were seeded in a white 96-well culture plate for adhesion cultures at 3*10⁵ cells per well, and PMA activator was added at concentrations indicated in the graph up to 150 µl in a well. All solutions were prepared in DMEM growth medium supplemented with 10% fetal bovine serum, 2 mM L-glutamine and 10 µg/ml gentamicin. The plate was incubated for 24 hours at 37 °C in a humid atmosphere in the presence of 5% CO₂. Following incubation, the wells were drained, and 100 µl of luciferin substrate with a lysing component was added, the plate was left in the dark for 10 minutes, after which the luminescence level was detected using a plate reader using the SparkControl V2.1 software package (Tecan, Austria).

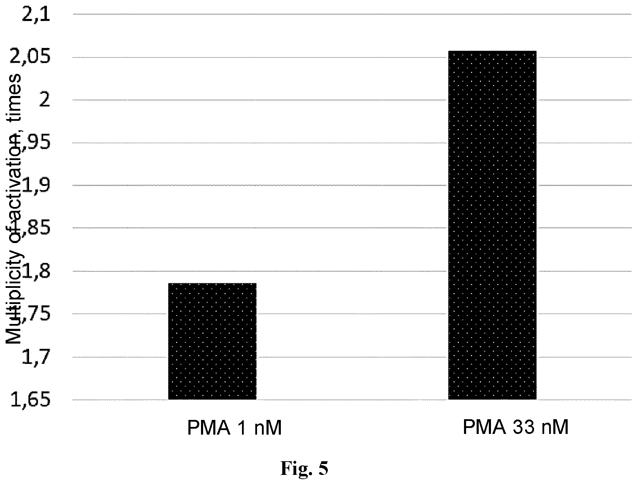

Thus, the optimal concentration of the PMA activator of 33nM was selected (Fig. 5).

### Example 4. Production of stable reporter cell line.

Important indicators for a reporter cell line are the multiplicity of activation in response to a test molecule, as well as the level of background activation.

A stable reporter cell line was produced using the HEK293 cell line (ATCC CRL-1573, USA) that was transfected with a plasmid vector comprising the reporter construct from Example 1. The resulting stable pool was cloned by limiting dilution cloning. To screen the clonal lines HEK293_STAT3-STAT5-AP1_FLuc, we conducted a luciferase expression activation test. The cells were seeded at 1*10⁵ cells per well in 96-well plates, and a 33 nM PMA activator was added up to the final volume of 150 µl in a well. All solutions were prepared in DMEM growth medium supplemented with 10% fetal bovine serum, 2 mM L-glutamine and 10 µg/ml gentamicin. The plate was incubated for 24 hours at 37 °C in a humid atmosphere in the presence of 5% CO₂. Following incubation, the wells were drained, and 100 µl of luciferin substrate with a lysing component was added, the plate was left in the dark for 10 minutes, after which the luminescence level was detected using a plate reader using the SparkControl V2.1 software package (Tecan, Austria).

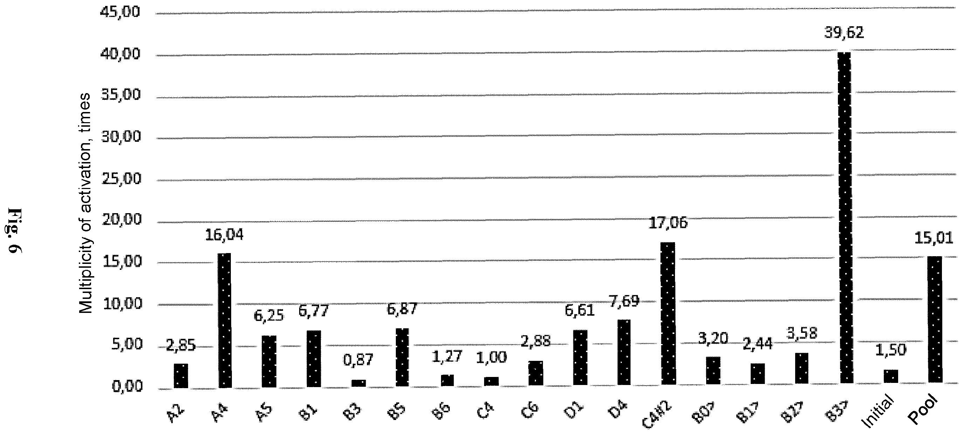

A reporter cell line was thus produced, that shows a high multiplicity of activation and a low level of background activation (Fig. 6).

### Example 5. Production of reporter line specific for IL10.

To evaluate the reporter line in a specific cell test, we expressed the IL-10 receptor onto the surface of the reporter cells. To this end, the HEK293_STAT3-STAT5-AP1_FLuc reporter cell line produced in Example 4 was transfected with plasmid vectors encoding IL-10 receptor subunits produced in Example 2. To screen clonal reporter lines expressing the IL10 receptor, we performed a luciferase expression activation test described in Example 3. Recombinant human IL10 at a concentration of 100 ng/ml was used as an activator.

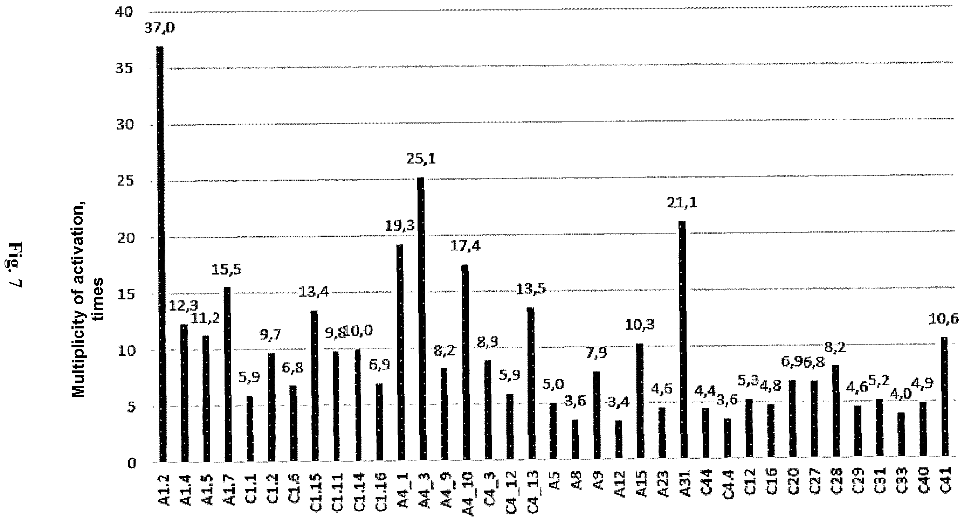

Screening and selection of clones based on multiplicity of activation resulted in a IL10-specific reporter line based on a universal line (Fig. 7).

### Example 6. IL10 activity determination test

Reporter lines are used to evaluate the activity of candidate medicinal products. We performed a luciferase expression activation test in response to various concentrations of IL10 activator shown in the graph. The test design has been described in Example 3.

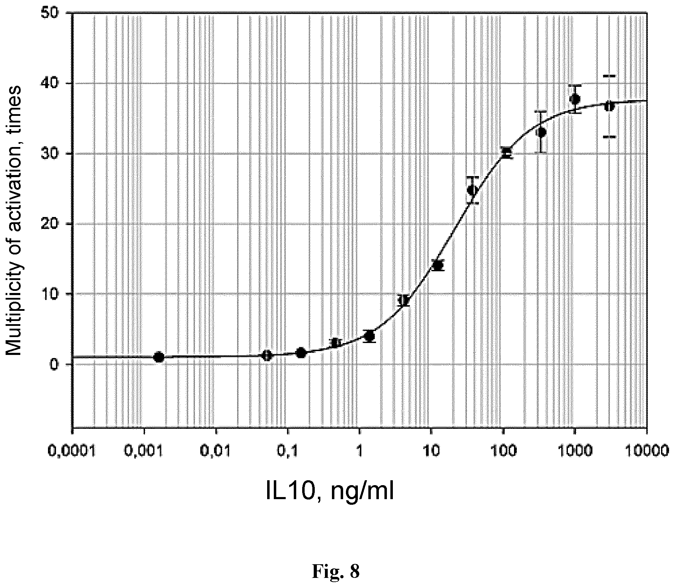

We eventually observed a dose-dependent response of the reporter cell line to a specific activator (Fig. 8).

### Example 7. Creation of reporter cell line HEK293_STAT3-STAT5-AP1_FLuc_AXL to evaluate AXL-mediated activation of intracellular signaling with Gas6 STAT3-STAT5-AP1 product, on the basis of universal reporter cell line HEK293_STAT3-STATS-AP1_FLuc.

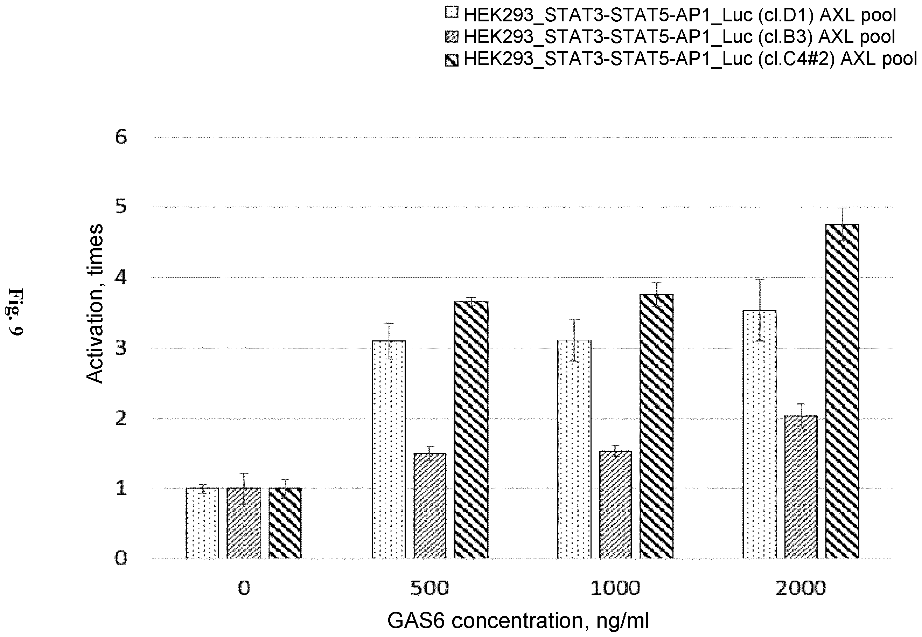

To generate the reporter cell line HEK293_STAT3-STAT5-AP1_FLuc_AXL, we used three clones of the universal reporter cell line HEK293_STAT3-STATS-AP1_FLuc: cl. D1, cl. B3 and cl. C4#2; on the basis of them, we produced, respectively, 3 pools of cells with an embedded constitutionally expressed AXL receptor gene. AXL-mediated activation of these pools was tested. The test was performed in a 96-well culture plate designed for luminescence assays. The suspension contained in each well 30,000 HEK293_STAT3-STAT5-AP1_FLuc_AXL cells and the AXL ligand GAS6 at concentrations shown in Fig. 9. The final volume of the cell suspension in a well was 100 µl, all components of the suspension were prepared in DMEM medium free of fetal bovine serum. After adding all the components, the plate was incubated for 16 hours at 37°C, 5%CO₂, and then, using a luminescence assay kit, we measured the luminescence intensity in the wells. The measurement was carried out using a plate reader. The activation level was calculated as the ratio of luminescence level for a given pool in the presence of GAS6 to luminescence level in the absence of GAS6.

### Example 8. Testing of various GAS6 products for AXL-mediated activation of STATS-STATS-API on reporter cell line HEK293_STAT3-STAT5-AP1_FLuc_AXL.

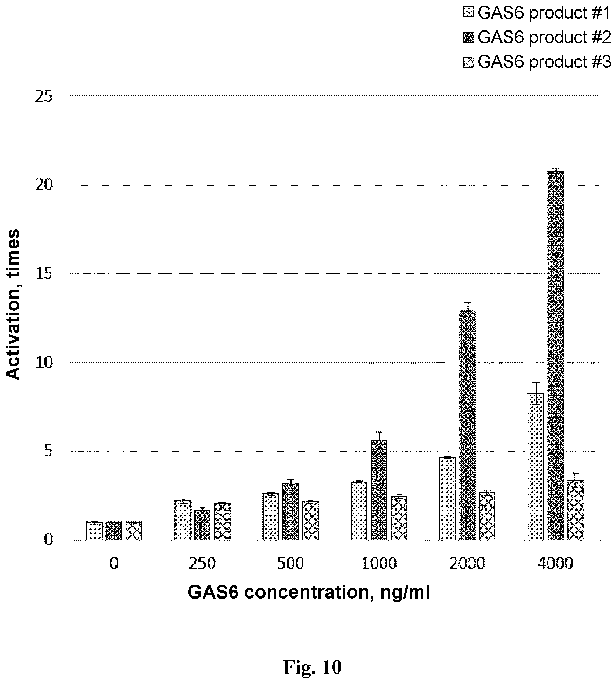

The test was performed similarly to the procedure described in Example 7 (in the presence of GAS6 at a concentration of 2 µg/ml); for activation, we used HEK293_STAT3-STAT5-AP1_FLuc_AXL (the pool produced on the basis of the cell line HEK293_STAT3-STAT5-AP1_FLuc : cl. D1, see Example 7) and various GAS6 preparations in the concentration shown in the graph (Fig. 10).

### Example 9. Analysis of AXL-mediated activation of HEK293_STAT3-STAT5-AP1_FLuc_AXL clones.

From pools produced on the basis of cell lines HEK293_STAT3-STAT5-AP1 FLuc: cl. D1, cl. B3 and cl. C4#2 (see Example 7), we selected individual clones that showed GAS6-dependent activation in the test (performed similarly to that in Example 7 in the presence of GAS6 at a concentration of 2 µg/ml). The clones that showed the highest level of activation were selected from a cell pool produced on the basis of cl. D1. Activation levels in one of the 96-well plates containing various clones of the given pool and the initial pool itself are shown in Table 1; it shows activations relative to non-activated cells, the H11 well corresponds to the pool. Gray indicates a well corresponding to the clone HEK293_STAT3-STAT5-AP1_FLuc_AXL (cl.13) used for further study.

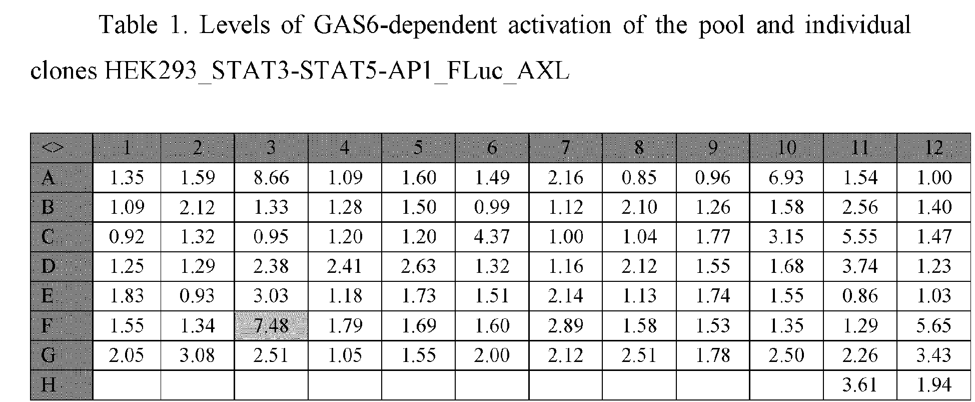

### Example 10. Study of activation of the reporter cell line HEK293_STAT3-STATS-AP1_FLuc_AXL as a function of incubation time in the presence of GAS6.

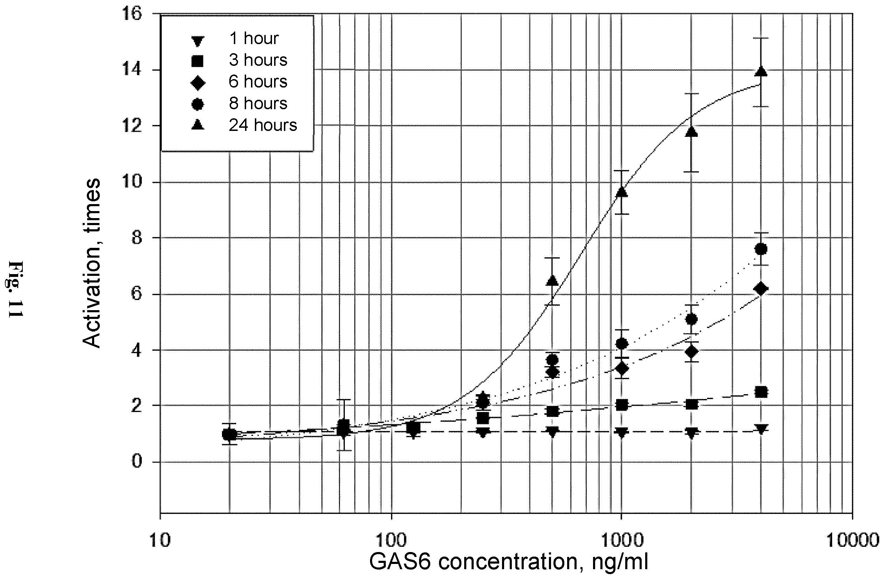

The test was performed similarly to the procedure described in Example 7, we used the cells of the reporter line HEK293_STAT3-STAT5-AP1_FLuc_AXL (cl.13) and GAS6 at a concentration of 2 µg/ml (Fig. 11) for activation; following adding all the components, the plate was incubated for the time shown in the graph, the luminescence intensity in the wells was then determined. The level of activation was calculated as the ratio of the luminescence value in the presence of said concentration of GAS6 to that in the absence of the GAS6 variant with the same incubation time. The highest level of activation was observed during 24-hour incubation.

### Example 11. Generation of a reporter cell line based on the naturally-occurring Du145 cell line to evaluate AXL-mediated activation of STAT3-STATS-API intracellular signaling.

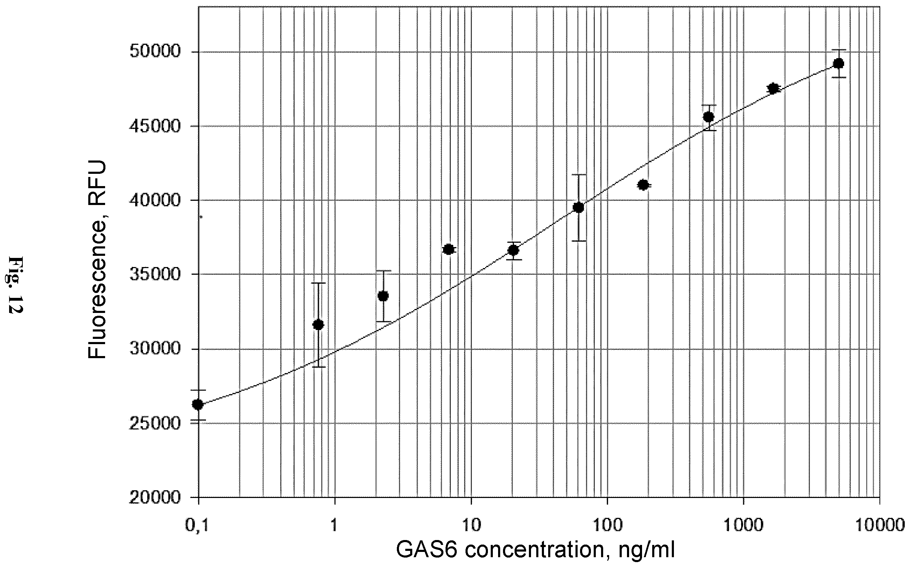

To test activation of Du145 cell proliferation with the AXL ligand GAS6 (Fig. 12), we used the Du145 cell line, a natural AXL expresser. The assay was performed in a 96-well culture plate for adhesion cultures. The suspension contained in each well 5,000 Du145 cells and the AXL ligand GAS6 at a concentration shown in the graph. The final volume of the cell suspension in a well was 100 µl, all components of the suspension were prepared in DMEM medium comprising 0.3% fetal bovine serum. Following adding all the components, the plate was incubated for 72 hours at 37 °C, 5% CO₂, and then 10 ml of Alamar Blue reagent was added to each well, the tablet was then shaken on an orbital shaker and incubated at 37 °C, 5% CO₂ for 6 hours. Fluorescence was measured at an excitation wavelength of 544 nm and emission wavelength of 590 nm using a plate reader.

We produced stable clones of Du145 cells comprising the STAT3-STAT5-AP1_FLuc gene encoding firefly luciferase under the control of STAT3-STAT5-AP1 promoter to generate a reporter cell line sensitive to GAS6-dependent activation of AXL signaling based on natural AXL-expressing cell line.

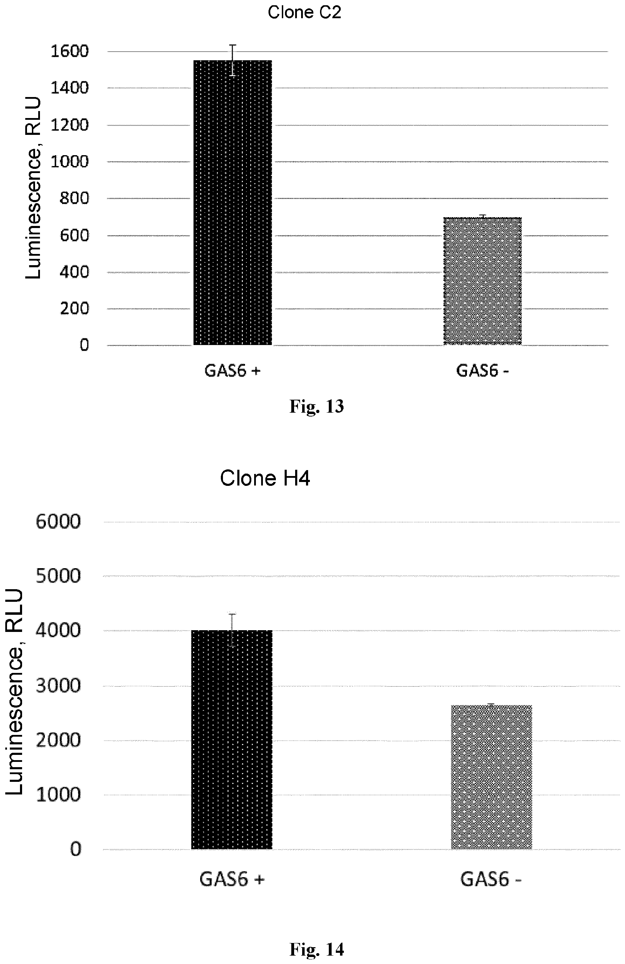

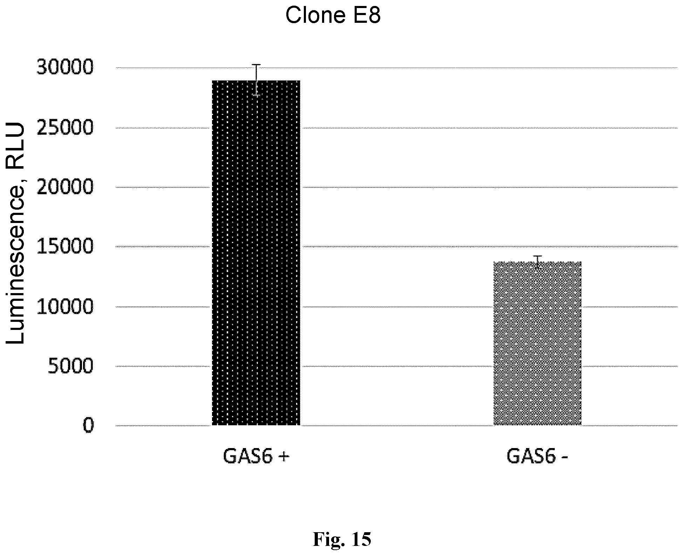

Clones that showed activation in the test were selected (Fig. 13-15). The test was carried out in a 96-well ELISA plate. A day before the experiment, the AXL ligand GAS6 was immobilized onto plastic: we prepared GAS6 solution at a concentration of 5 µg/ml in DPBS, and 100 µl were added into each plate well. The plate was incubated for 16 hours at 37 °C with 5% CO₂. Next, we added a suspension comprising 50,000 Du145_STAT3-STAT5-AP1_FLuc cells. The final volume of cell suspension was 100 µl per well. Following adding the cell suspension, the plate was incubated for 16 hours at 37°C, 5%CO₂, and then, using a luminescence assay kit, we measured the luminescence intensity in the wells. The measurement was carried out using a plate reader.

### Example 12. Creation of reporter cell line HEK293_AXL_STAT3-STAT5-AP1_FLuc based on AXL-overexpressing cell line HEK-293-AXL to evaluate AXL-mediated activation of STAT3-STAT5-AP1 intracellular signaling.

To create the reporter cell line HEK293_AXL_STAT3-STAT5-AP1_FLuc, we used the cell line HEK293 AXL, an AXL overexpresser, on the basis of which we produced a pool of cells comprising a gene encoding firefly luciferase under the control of STAT3-STAT5-AP1 promoter. Next, were selected individual clones from this pool, which showed activation in the test (Fig. 16). The test was performed in a similar fashion to the procedure described in Example 7, at a concentration of GAS6 of 2 µg/ml. The level of activation was calculated as the ratio of the luminescence value for the given clone in the presence of GAS6 to that in the absence of GAS6.

### Example 13. Study of activation of the reporter cell line HEK293_AXL_STAT3-STAT5-AP1 as a function of cell count.

The test was performed in a similar fashion to the procedure described in Example, 7 at a concentration of GAS6 of 2 µg/ml, cells of the reporter line HEK293_AXL_STAT3-STAT5-AP1 were used for activation, with the cell count per well of a 96-well plate as shown in the graph (Fig. 17). The highest level of activation was observed when the well contained 25,000-50,000 cells of the reporter line.

## Claims

1. An inducible promoter comprising binding sites of transcription factors STAT3, STATS and AP-1 and a minimal promoter.

2. The inducible promoter according to claim 1, wherein the binding site of the transcription factor STATS is a nucleotide sequence selected from a group of nucleotide sequences including SEQ ID NO: 1, SEQ ID NO: 2 or SEQ ID NO: 3.

3. The inducible promoter according to claim 1, wherein the binding site of the transcription factor STATS is a nucleotide sequence selected from a group of nucleotide sequences including SEQ ID NO: 4, SEQ ID NO: 5 or SEQ ID NO: 6.

4. The inducible promoter according to claim 1, wherein the binding site of the AP-1 family transcription factor is a nucleotide sequence selected from a group of nucleotide sequences including SEQ ID NO: 7, SEQ ID NO: 8 or SEQ ID NO: 9.

5. The inducible promoter according to claim 1, wherein the binding sites of transcription factors STAT3, STATS and AP-1 are arranged from 5' end to 3' end one after the other in the following order: STAT3-STAT5-AP-1.

6. The inducible promoter according to claim 5, wherein the binding sites of the transcription factors STAT3, STATS and AP-1 are arranged from 5' end to 3' end one after the other in the following order: STAT3-STAT5-AP-1, and comprise a nucleotide sequence selected from a group of nucleotide sequences including SEQ ID NO: 10, SEQ ID NO: 11 or SEQ ID NO: 12.

7. The inducible promoter according to claim 1, wherein the binding sites of transcription factors STAT3, STATS and AP-1 comprise the nucleotide sequence of SEQ ID NO: 13.

8. The inducible promoter according to claim 1, wherein the minimal promoter comprises a TATA box.

9. The inducible promoter according to claim 1, wherein the minimal promoter comprises the nucleotide sequence of SEQ ID NO: 14.

10. An inducible expression vector comprising, in 5' end to 3' end direction, the inducible promoter according to any one of claims 1 to 9 and a reporter gene.

11. The inducible expression vector according to claim 10, wherein the reporter gene is a gene of firefly luciferase protein.

12. The inducible expression vector according to claim 11, wherein the gene of firefly luciferase protein comprises the nucleotide sequence of SEQ ID NO: 15.

13. The inducible expression vector according to claim 10, comprising the nucleotide sequence of SEQ ID NO: 16.

14. A method for producing a host cell for analyzing the activity of target protein, comprising transforming the cell with the inducible expression vector according to any one of claims 10 to 13.

15. A host cell for analyzing the activity of target protein comprising the inducible promoter according to any one of claims 1 to 9 and a reporter gene.

16. The host cell according to claim 15, wherein the reporter gene is a gene of firefly luciferase protein.

17. The host cell according to claim 16, wherein the gene of firefly luciferase protein comprises the nucleotide sequence of SEQ ID NO: 15.

18. The host cell according to claim 15, comprising the nucleotide sequence of SEQ ID NO: 16.

19. The host cell according to claim 15, wherein the target protein is a receptor ligand.

20. The host cell according to claim 19, wherein the receptor ligand is cytokine.
